# EUROPEAN PATENT APPLICATION

(11) **EP 4 417 976 A1**
(43) Date of publication of application: **21.08.2024**
(21) Application number: 22882369.6
(22) Date of filing: 22.07.2022
(51) Int. Cl.: G01N 35/00, G01N 35/10, C12M 1/00

(54) **SYSTEM FOR MACROMOLECULE EXTRACTION**

(30) Priority: 21.10.2021 CN 202111225397
(71) Applicant: Innovel Intelligent Technology Co., Ltd., Suzhou, Jiangsu 215000 (CN)
(72) Inventor: CHEN, Hao, Suzhou, Jiangsu 215000 (CN); YANG, Kailin, Suzhou, Jiangsu 215000 (CN); ZHI, Yan, Shanghai 200131 (CN); CHEN, Xiaoyue, Shanghai 200131 (CN)
(74) Representative: Arnold & Siedsma
(86) International application number: PCT/CN2022/107404
(87) International publication number: WO 2023/065747

(57) **Abstract**

The present disclosure relates to a system for the extraction of macromolecules comprising: one or more incubators, the incubators for culturing a biological sample to provide a base sample for screening for the detection of the macromolecule; a sample operating system, the sample operating system for performing predetermined operations on the biological sample cultured in the incubator; a sample detection platform, the sample detection platform for detecting the biological sample for screening; and a transfer platform, the transfer platform for transferring the biological samples between the incubator and the sample operating system and/or between the sample operating system and the sample detection platform. The system according to the present disclosure not only quantitatively increases the throughput of extraction of macromolecules while quantitatively reducing the number of operators, but also greatly improves the stability, homogeneity, and reproducibility of the process by standardizing operations.

## Description

### Technical Field

The present disclosure relates generally to the technical field of macromolecules. More particularly, the present disclosure relates to a system for the extraction of macromolecules.

### Background of Invention

The biopharmaceutical industry is in a period of rapid development, with large molecule biopharmaceuticals being put into large-scale use. The research and development of new drugs, especially the selection of effective target products at the early stage, has become the key to the sustained competitiveness of an enterprise's product line. The traditional manual production method is difficult to meet the upcoming large-scale production of large molecule biopharmaceuticals in the research and development stage and the selection of target products.

The traditional manual production method is suitable for single and small batch production. To realize industrial-scale production with traditional manual production methods requires: 1) a large number of qualified operators, 2) a large variety of various types of instruments and equipment to operate manually, and 3) a large area of clean space. The following problems exist: 1) low utilization of equipment and clean space, 2) the quality of the product is affected by the technical level of the operator, 3) low flexibility, which makes it difficult to realize rapid process iteration and scale-up, 4) low efficiency of production and target product selection, which makes it difficult to synthesize, produce, and select the effective product in large-scale potential samples.

### Summary of Invention

It is one of the objects of the present disclosure to solve one or more of the above problems and to realize other additional advantages.

In a first aspect of the present disclosure, there is provided a system for the extraction of macromolecules, comprising: at least one incubator for culturing a biological sample to provide a base sample for the extraction of the macromolecules; a sample operating system for performing predetermined operations on the biological sample cultured by the incubator to extract macromolecules from the base sample; a transfer platform for transferring the biological sample between the incubator and the sample operating system.

According to an embodiment of the present disclosure, the transfer platform comprises a robot.

According to an embodiment of the present disclosure, the robot is provided on a ground rail or an air rail.

According to an embodiment of the present disclosure, the transfer platform comprises a transit sliding table, the robot being capable of transferring the biological sample from the incubator to the transit sliding table, the transit sliding table being configured for transferring the biological sample to the sample operating system.

According to an embodiment of the present disclosure, the transit sliding table is capable of transferring biological samples in batch.

According to an embodiment of the present disclosure, a plurality of incubators is provided, the plurality of incubators being arranged on both sides of the transfer platform.

According to an embodiment of the present disclosure, the incubator comprises a housing and a culture assembly accommodated within the housing, the culture assembly being provided on an automatic sliding table element to enable the culture assembly to be slid out of or into the housing in a controlled manner.

According to an embodiment of the present disclosure, the incubator is configured to conduct automatic temperature control and humidity control of the biological sample, wherein the internal temperature of the incubator is controlled at 15 to 40°C, and the humidity thereof is controlled at 70 to 95%, for amplification culture.

According to an embodiment of the present disclosure, the incubator comprises a heating membrane, wherein the heating membrane automatically heats the incubator and controls the internal temperature thereof at 15 to 40°C.

According to an embodiment of the present disclosure, the incubator comprises a humidification unit, the humidification unit comprising an automatic water supply unit and a nebulizer, the automatic water supply unit being configured to supply water to the incubator in a controlled manner, and the nebulizer being configured to control the humidity of the incubator by nebulizing the water from the automatic water supply unit.

According to an embodiment of the present disclosure, the incubator comprises a refrigeration compressor, the refrigeration compressor being configured to control the temperature of the biological samples at 2 to 8°C when sedimentation enrichment of the biological sample is required.

According to an embodiment of the present disclosure, a portion of the incubators is configured to carry out an amplification culture of the biological samples, and another portion of the incubators is configured to simultaneously carry out a sedimentation enrichment of the biological samples.

According to an embodiment of the present disclosure, the sample operating system is arranged in a controlled laminar flow environment such that the internal environment of the sample operating system is isolated from its external environment.

According to an embodiment of the present disclosure, the sample operating system is provided with a laminar flow unit, the laminar flow unit being configured to form the laminar flow environment inside the sample operating system using a pressure difference.

According to an embodiment of the present disclosure, the laminar flow unit comprises a laminar flow air supply unit and a laminar flow air return unit, the laminar flow air supply unit being arranged at the top of the sample operating system and the laminar flow air return unit being arranged at the bottom of the sample operating system.

According to an embodiment of the present disclosure, the sample operating system comprises an operating table surface, on which two sets of sample operating assemblies are mounted, the two sets of sample operating assemblies being arranged mirror-symmetrically about a central axis of the operating table surface on one side of the operating table surface respectively, and the two sets of sample operating assemblies being configured to be capable of carrying out both independent operation and parallel operation.

According to an embodiment of the present disclosure, the sample operating assembly comprises at least one of the following devices: a code-scanning unit, a centrifuge tube shaking unit, a shake-flask shaking unit, a centrifuge tube uncapping unit, a shake-flask uncapping unit, a magnetic bead sorting unit, a centrifuge unit, a pipette carrier table, and a multi-degree-of-freedom robot.

According to an embodiment of the present disclosure, a pipetting unit and a cryopreservation tube uncapping unit are further mounted on the operating table surface, the pipetting unit and the cryopreservation tube uncapping unit being arranged on a central axis of the operating table surface.

According to an embodiment of the present disclosure, the sample operating system is configured to perform at least one of the following operations: scanning, uncapping, shaking, centrifugal separation, magnetic bead sorting, pipetting, pumping and heating.

According to an embodiment of the present disclosure, the multi-degree-of-freedom robot is mounted on a linear motor module, the linear motor module comprising a transverse beam extending in a horizontal direction and a linear motor mounted on the transverse beam and driving the multi-degree-of-freedom robot in the direction of extension of the transverse beam, the multi-degree-of-freedom robot being capable of moving on the transverse beam such that the range of action of the multi-degree-of-freedom robot is capable of substantially covering the operating area of each device of the sample operating assembly.

According to an embodiment of the present disclosure, the sample operating system further comprises a pumping unit, the pumping unit comprising a liquid storage unit for storing liquids required for the process, a pump body panel for selecting a liquid pumping channel, a pumping actuation module for pumping liquids into consumables, and a motion module for moving the pumping actuation module.

According to an embodiment of the present disclosure, the pump body panel is configured for selecting a liquid pumping channel among a plurality of liquid pumping channels, the pumping actuation module being able to move to different positions by the motion module and to perform a liquid pumping operation using the selected liquid pumping channel.

According to an embodiment of the present disclosure, the liquid storage unit comprises at least one liquid storage container.

According to an embodiment of the present disclosure, a weighing unit can be installed under the liquid storage container, by which the liquid remaining in the liquid storage container can be weighed.

According to an embodiment of the present disclosure, the pumping actuation module is configured as a centrifuge tube pumping device or a shake-flask pumping device.

According to an embodiment of the present disclosure, the centrifuge tube shaking unit is configured as a centrifuge tube oscillating and over-turning unit which is configured to rotate the centrifuge tube at an angle at 0 degree to 360 degrees for mixing, wherein at least one of the following parameters can be controlled during the mixing process: rotation speed, number of rotation cycles, and direction of rotation.

According to an embodiment of the present disclosure, the centrifuge tube uncapping unit comprises a centrifuge tube loading linear module and a centrifuge tube uncapping machine working head, the centrifuge tube loading linear module being configured to transport a centrifuge tube carrier tray underneath the centrifuge tube uncapping machine working head after the multi-degree-of-freedom robot has placed the centrifuge tube carrier tray with centrifuge tubes on the centrifuge tube loading linear module.

According to an embodiment of the present disclosure, the centrifuge tube loading linear module is assigned with a centrifuge tube automatic positioning and alignment mechanism, the centrifuge tube automatic positioning and alignment mechanism being configured to position and clamp the centrifuge tube carrier tray.

According to an embodiment of the present disclosure, the magnetic bead sorting unit comprises a magnetic bead sorting rack for performing magnetic bead adsorption and a magnetic bead pouring rack for dumping waste supernatant.

According to an embodiment of the present disclosure, the magnetic bead adsorption operation and the waste supernatant pouring operation can be performed simultaneously.

According to an embodiment of the present disclosure, the cryopreservation tube uncapping unit comprises a cryopreservation tube loading linear module and a cryopreservation tube uncapping machine working head, the cryopreservation tube loading linear module being configured to transport a cryopreservation tube carrier tray underneath the cryopreservation tube uncapping machine working head after the multi-degree-of-freedom robot has placed the cryopreservation tube carrier tray on the cryopreservation tube loading linear module.

According to an embodiment of the present disclosure, the cryopreservation tube loading linear module is assigned with a cryopreservation tube automatic positioning and alignment mechanism, the cryopreservation tube automatic positioning and alignment mechanism being configured to position and clamp the cryopreservation tube carrier tray.

According to an embodiment of the present disclosure, the pipetting unit is configured to transfer the liquid required in the process to a consumable placed on the pipette carrier table to perform the corresponding operation.

According to an embodiment of the present disclosure, the pipetting unit comprises a pipetting robot as well as a pipetting module, the pipetting module being configured to be capable of moving independently within the pipetting unit along a Z-axis, wherein the Z-axis is perpendicular to a horizontal plane.

According to an embodiment of the present disclosure, the pipetting robot is configured as a SCARA robot.

According to an embodiment of the present disclosure, the sample operating system further comprises an operation carrier and a consumable carrier sliding table for carrying the operation carrier, the consumable carrier sliding table being configured to be capable of sliding out of and into the sample operating system in a controlled manner so as to facilitate loading or unloading of consumables on the operation carrier.

According to an embodiment of the present disclosure, the operating table surface together with the sample operating assembly mounted on the operating table surface is arranged in an intermediate cabin.

According to an embodiment of the present disclosure, the liquid storage unit and the pump body panel are arranged in a bottom bin.

According to an embodiment of the present disclosure. The system for the extraction of macromolecules further comprises an automated control system, the automated control system being configured to control operations in the incubator, the sample operating system, and the transfer platform.

In a second aspect of the present disclosure, there is provided a system suitable for automated production of extraction of macromolecules, comprising: a top cabin, an intermediate cabin, a bottom cabin, a temperature-controlled incubator, a robot and a rail, a sample operating system and an electrical control system, the top cabin comprising a laminar flow air intake and a robot rail, the intermediate cabin comprising a code-scanning unit, an uncapping unit, a shaking unit, a centrifuge, a magnetic bead sorting unit, a pipetting unit, a pump-liquid storage unit and a pump body unit, a heating unit, an electrically operated door and a manual door module, the bottom cabin comprising a liquid storage cabin, a centrifugal equipment storage cabin, a pump valve control cabin, an air return blower and a cable liquid circuit.

As a preferred embodiment of the present invention, an equipment frame unit is provided within the temperature-controlled incubator, wherein a shaking bed mechanism driving unit is provided on the lower side of the interior of the temperature-controlled incubator, wherein an automatic sliding table unit is provided in the interior of the temperature-controlled incubator and located on the upper side of the shaking bed mechanism driving unit, wherein a shake-flask carrier tray unit is provided on the upper side of the automatic sliding table unit, wherein an equipment lighting/purification/sanitization unit is provided on the upper side of the interior of the temperature-controlled incubator, wherein an intelligent integrated control unit is provided on the right rear side of the temperature-controlled incubator, wherein an equipment operation and display area is provided on the right side of the temperature-controlled incubator, wherein an automatic sealing door unit is provided on the right side of the end of the temperature-controlled incubator.

As a preferred solution of the present invention, the sample operating system includes a loading sliding table, a pipette carrier table, a cryopreservation tube uncapping unit, a pipetting robot and module, a centrifuge tube uncapping unit, a centrifuge, a magnetic bead sorting unit, a centrifugal over-turning unit, a centrifuge tube oscillating unit, a manual loading table, a handling robot, and an automatic loading table for shake-flasks to open the caps.

As a preferred solution of the present invention, the pump liquid storage and pump body unit includes large capacity liquid storage, multi-channel pump body panel, pump liquid execution unit linear motion module, pumping execution module.

As a preferred solution of the present invention, the cryopreservation tube uncapping unit and centrifuge tube uncapping unit both include loading linear module, uncapping machine working head, and automatic positioning and alignment mechanism.

As a preferred embodiment of the present invention, the robot independently moves the pipetting unit along the Z-axis, and is mounted on a SCARA robot platform.

As a preferred embodiment of the present invention, the electrical control system includes temperature sensors, weight sensors, differential pressure sensors, photoelectric sensors, cameras, code readers, I/O acquisition modules, industrial control computer and drive modules.

Additional and/or other aspects and advantages of the present disclosure will be set forth in the following description, or will be apparent from the description or can be learned by practicing the present disclosure. The various technical features of the present disclosure may be combined in any way, as long as they do not contradict each other.

### Brief Description of Drawings

The above mentioned features and advantages and other features and advantages of the present disclosure, as well as the manner of realizing them, will become more apparent in conjunction with the accompanying drawings and with reference to the following detailed description of specific embodiments of the present disclosure. In the accompanying drawings:
FIG. 1 is a front view of the basic structure of a system for the extraction of macromolecules according to an embodiment of the present disclosure;
FIG. 2 is a schematic diagram of a fully automated incubator unit according to an embodiment of the present disclosure;
FIG. 3 is a system diagram of an incubator array and a system for operating a sample transfer robot, according to an embodiment of the present disclosure;
FIG. 4 is a top view of a connection between an incubator array and a sample operating system according to an embodiment of the present disclosure;
FIG. 5 is a plan view of a sample operating system according to an embodiment of the present disclosure;
FIG. 6 is a schematic view of an array of pump fluid storage and pump units according to an embodiment of the present disclosure;
FIG. 7 is a schematic diagram of a centrifuge tube oscillating and over-turning unit according to an embodiment of the present disclosure;
FIG. 8 is a schematic diagram of a centrifuge lid opening unit according to an embodiment of the present disclosure;
FIG. 9 is a schematic diagram of a magnetic bead sorting unit according to an embodiment of the present disclosure;
FIG. 10 is a schematic diagram of a cryopreservation tube uncapping unit according to an embodiment of the present disclosure;
FIG. 11 is a schematic diagram of a pipetting unit according to an embodiment of the present disclosure;
FIG. 12 is a perspective view of the basic structure of a sample operating system according to an embodiment of the present disclosure;
FIG. 13 is another perspective view of the basic structure of a sample operating system according to an embodiment of the present disclosure;
FIG. 14 is a schematic diagram of a sample operating process according to an embodiment of the present disclosure;
FIG. 15 is a schematic view of a basic automated control architecture according to an embodiment of the present disclosure;
FIG. 16 is a schematic diagram of a software architecture for an intelligent extraction of macromolecules system according to an embodiment of the present disclosure.

In the accompanying drawings, corresponding attachment marks indicate corresponding components. The examples described herein are used to illustrate exemplary aspects of the present disclosure, and these examples should not be construed to limit the scope of the present disclosure in any way.

### Detailed Description of Drawings

The present disclosure will be described below with reference to the accompanying drawings, which illustrate several embodiments of the present disclosure. It should be understood, however, that the present disclosure can be presented in a number of different ways and is not limited to the embodiments described below; in fact, the embodiments described below are intended to make the disclosure of the present disclosure more complete and to adequately illustrate to those skilled in the art the scope of protection of the present disclosure. It should also be understood that the embodiments disclosed herein are capable of being combined in a variety of ways to provide more additional embodiments.

For descriptive purposes, the terms "up", "down", "left", "right", "vertical", "horizontal", "top", "bottom", " transverse", "longitudinal", and their derivatives are all related to the orientation in the accompanying drawings of the present disclosure. It should be understood, however, that the present disclosure may employ a variety of alternative variations unless expressly stated to the contrary. For example, a feature previously described as being "below" other features may be described as being "above" other features when the device in the accompanying drawings is turned upside down. The device may also be oriented in other ways (rotated 90 degrees or in other orientations), and the relative spatial relationships will be explained accordingly.

The singular forms "one", "the" and "the" used in the description include the plural form, unless clearly indicated. The terms "includes", "comprises" and "contains" are used in the description to indicate the presence of the claimed feature, but do not exclude the presence of one or more other features. The term "and/or" as used in the description includes any and all combinations of one or more of the relevant listed items.

In the description, when an element is the to be disposed "on" another element, "attached" to another element, "connected" to another element, "coupled" to another element, "in contact" with another element, etc., the element may be directly disposed on another element, attached to another element, connected to another element, coupled to another element, or in contact with another element, or there may be an intermediate element. By contrast, when an element is the to be "directly" disposed "on" another element, "directly attached" to another element, "directly connected" to another element, "directly coupled" to another element or "directly in contact" with another element, there will be no intermediate element. In the description, a feature arranged "adjacent" to another feature may mean that a feature has a portion that overlaps an adjacent feature or is located above or below an adjacent feature.

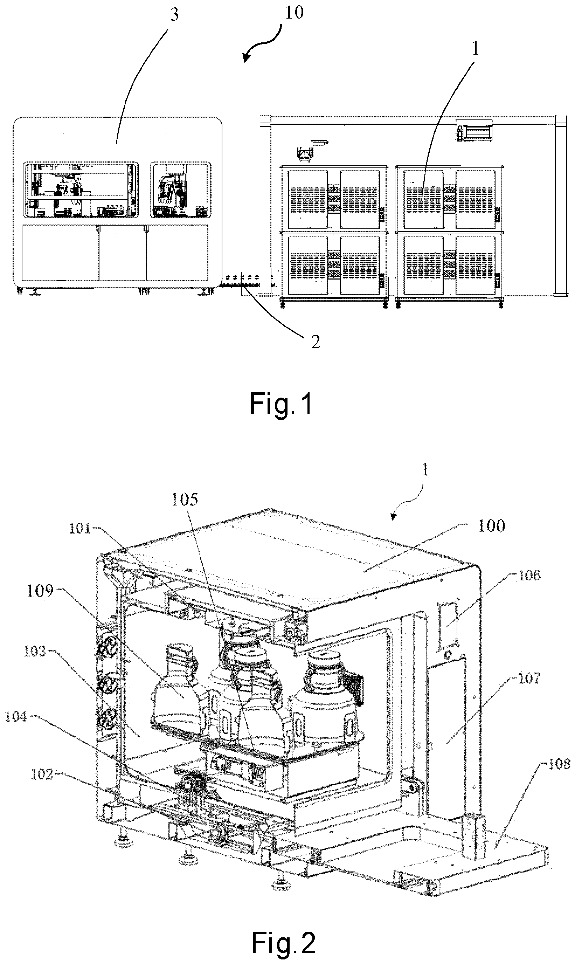

Referring first to FIG. 1, a system for the extraction of macromolecules according to an embodiment of the present disclosure is illustrated. The system 10 may comprise: at least one incubator 1, the incubator 1 being used to culture a biological sample to provide a base sample for the extraction of macromolecules; a sample operating system 3, the sample operating system 3 being used to carry out predetermined operations on the biological sample cultured in the incubator 1 (e.g., operations related to the extraction for macromolecules, such as uncapping, shaking, centrifugation, magnetic bead sorting, pipetting, pumping, etc.) for extracting macromolecules from the base sample; and a transfer platform 2, the transfer platform 2 being used to transfer the biological sample between the incubator 1 and the sample operating system 3. In an embodiment according to the present disclosure, the transfer platform 2 may be configured as a culture material transfer platform, which culture material transfer platform is used to transfer the cultured biological sample between the incubator 1 and the sample operating system 3. For example, the culture material transfer platform may transfer the biological samples cultured by the incubator 1 to the sample operating system 3 for the sample operating system 3 to carry out the corresponding operations, and may also transfer the biological samples from the sample operating system 3 back to the incubator 1 for further culturing. The plurality of incubators 1 may be configured as an array of incubators.

In an embodiment according to the present disclosure, the transfer platform 2 may comprise a robot 210 (also known as a robot arm), wherein the robot 210 may be utilized to realize the transfer of the biological samples between the incubator 1 and the sample operating system 3. In an embodiment according to the present disclosure, the robot 210 may be provided on a ground rail or an air rail. Thereby, the robot 210 can be moved on the ground rail and/or the air rail to perform the transfer of the biological samples over a larger area. By using the high-speed, high degree-of-freedom robot automated material handling module, loading and unloading, transferring, etc. of a wide variety of process materials (shake-flasks, centrifuge tubes, centrifuge tube carriers, cryopreservation tube trays, 96-well plates, tip box, magnetic bead carriers, workpieces, etc.) can be achieved, and an efficient connection and transfer between the incubator arrays and the sample operating system 3 can be realized.

The incubator 1 and the sample operating system 3 and the transfer platform 2 of the system 10 will be described in detail below, respectively.

Referring to FIG. 2, a specific structure of the incubator 1 according to an embodiment of the present disclosure is shown. The incubator 1 may include a housing 100 and a culture assembly accommodated within the housing 100. The culture assembly may include one or more shake-flasks 109 for holding biological samples. The one or more shake-flasks 109 may be placed on a shake-flask carrier tray assembly 105. The shake-flask carrier tray assembly 105 may be placed on an automatic sliding table unit 104 to allow the shake-flask carrier tray assembly 105 and the shake-flasks 109 it carries to be slid out of or into the housing 100 in a controlled manner when needed. The shake-flask carrier tray assembly 105 and the automatic sliding table unit 104 may be located within the frame unit 103. An oscillating unit (e.g., a shaking bed mechanism or shaking bed mechanism-type drive unit) 102 may be provided below the frame unit 103 so that the shake-flask carrier tray assembly 105 and the shake-flasks 109 it carries may be oscillated when needed. The interior of the incubator 1 may also be provided with an equipment lighting/purification/sanitization unit 101. The equipment lighting/purification/sanitization unit 101 may be provided in the interior of the incubator 1 in an upper side position of the incubator. A suitable equipment lighting/purification/sanitization unit 101 may be a UV irradiation unit.

In an embodiment according to the present disclosure, the incubator 1 may be an intelligent incubator. To this end, the incubator 1 is provided with an intelligent integrated control system 107 to realize intelligent control of the incubator 1. For example, the integrated control system 107 may control real-time oscillation of the oscillating unit 102, sliding in and out of the automatic sliding table unit 104, opening and closing of the equipment lighting/purification/sanitization unit, and the like. To facilitate operation of the intelligent integrated control system 107, the incubator 1 may also comprise an equipment operation and display area 106. The equipment operation and display area 106 may comprise input/output components, such as a touch screen, to provide a human-machine interaction interface. In an embodiment according to the present disclosure, the incubator 1 may further comprise an automatic sealing door unit 108. The automatic sealing door unit 108 may be automatically opened or closed under the control of the integrated control system 107 to open or close the incubator 1. All of these contribute to the automated docking of the incubator 1 with the culture material transfer platform. In an embodiment according to the present disclosure, the intelligent network unit may also be utilized to transmit commands to the integrated control system 107 via software installed in a remote terminal (e.g., a computer, a smartphone, or other controller, etc.) to achieve remote control of the incubator 1.

In an embodiment according to the present disclosure, the incubator 1 may be configured to be capable of automated temperature and humidity controlled culture of biological samples. For example, the incubator 1 can achieve a temperature control of 15 to 40°C and/or a humidity control of 70 to 95%. The temperature control of the incubator 1 can be achieved using a heating membrane and a thermal insulation material. In this embodiment, the heating membrane may be made of a film heating material for automatically heating the incubator 1 to keep it in a predetermined temperature range, and the thermal insulation material may be used to reduce the temperature transfer between the internal space of the incubator 1 and the outside world. In order to realize humidity control, the incubator 1 may comprise a humidification unit. The humidification unit may comprise an automatic water supply unit and a nebulizer. The nebulizer may control the humidity of the incubator 1 by nebulizing water from the automatic water supply unit. This is in contrast to conventional incubators which require an operator to set an amount of water inside them to maintain humidity. The nebulizer can be configured as an ultrasonic nebulizer. The nebulizer is capable of being started or stopped as needed under the control of the integrated control system 107 to effectively control the humidity within the incubator 1.

In an embodiment according to the present disclosure, the incubator 1 may also be configured to enable automated cryogenic sedimentation enrichment of biological samples. A refrigeration compressor may be provided within the incubator 1. The refrigeration compressor is configured to control the temperature of the biological samples at 2 to 8°C when sedimentation enrichment of biological samples is required, thereby realizing cryogenic sedimentation of the biological samples.

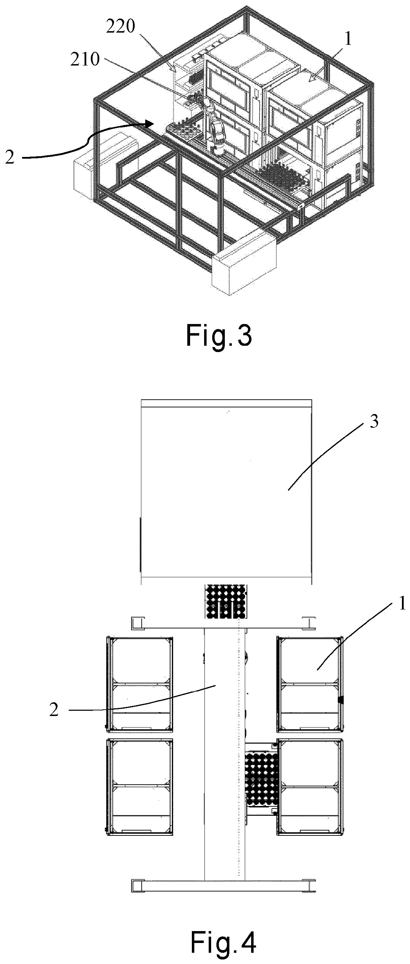

Referring to FIGS. 3 and 4, a system diagram of the incubator array and the operation sample transfer robot and a top view of the connection of the incubator array to the sample operating system 3 are shown.

As described above, the transfer platform 2 can transfer the biological samples cultured by the incubator 1 to the sample operating system 3 and also transfer the biological samples from the sample operating system 3 back to the incubator 1. When the transfer platform 2 transfers the biological samples from the incubator 1 to the sample operating system 3, firstly, under the control of the integrated control system 107, the automatic sealing door unit 108 is automatically opened. Then the automatic sliding table unit 104 slides out to allow the shake-flask carrier tray assembly 105 and the shake-flasks 109 it carries to slide out of the housing 100 of the incubator 1. Then a robot 210 (e.g., a robot arm) for transferring transfers the shake-flask carrier tray assembly 105 carrying the shake-flasks to the transfer platform 2. In an embodiment according to the present disclosure, the shake-flask carrier tray assembly 105 may be transferred by the robot into a transit transport table 220 of the transfer platform 2 for temporary storage, or intermediate storage, of the shake-flask carrier tray assembly 105. When all of the equipment available for operation in the sample operating system 3 is occupied, temporary storage of the shake-flask carrier tray assembly 105 to be operated is required if necessary, at this time the transit transport table 220 may assume the role of that temporary storage. When the sample operating system 3 is again available for the shake-flask carrier tray assembly 105 to be operated, the shake-flask carrier tray assembly 105 is then transferred by the robot 210 from the transit transport table 220 to the sample operating system 3. In an embodiment according to the present disclosure, when the sample operating system 3 is available, the shake-flask carrier tray assembly 105 may also be transferred to the transfer platform 2 via the robot 210 after being slid out of the housing 100 of the incubator 1 and transferred directly by the transfer platform 2 to the sample operating system 3. Thus, the biological samples can be transferred to the sample operating system 3 in batch via the transfer platform 2 to complete the loading into the interior of the sample operating system 3. In the sample operating system 3, the sample operating system 3 performs sample operations on the biological samples and completes data collection and analysis. When the biological samples need to be cultured again, the biological samples can be transferred again from the sample operating system 3 to the transfer platform 2. In an embodiment according to the present disclosure, after the shake-flask carrier tray assembly 105 has emerged from the sample operating system 3, if there is no incubator 1 available, the robot 210 may be utilized to transfer the shake-flask carrier tray assembly 105 to the transit transport table 220 for temporary storage. When an incubator 1 is available, the robot 210 can be utilized to transfer the shake-flask carrier tray assembly 105 from the transit transport table 220 of the transfer platform 2 to an available incubator 1 again. In an embodiment according to the present disclosure, the shake-flask carrier tray assembly 105 may also be transferred directly to the transfer platform 2 after emerging from the sample operating system 3. The shake-flask carrier tray assembly 105 is transferred from the transfer platform 2 to the automatic sliding table unit 104. The automatic sealing door unit 108 is automatically opened under the control of the integrated control system 107. The automatic sliding table unit 104 is then slid in to allow the shake-flask carrier tray assembly 105 to be transferred again into the incubator 1.

After the incubator array acquires the culture consumables (e.g., shake-flasks, etc.), they are first placed in batch on the transit sliding table, and then transferred in batch to the sample operating system 3 after a single batch has been placed, which can greatly improve the transfer efficiency. After the shake-flasks have completed the replenishment/exchange operation, the batch is transferred to the sliding table and uniformly transferred back to the incubator array.

As shown in FIG. 4, in the case where a plurality of incubators 1 are provided, the plurality of incubator 1 may be arranged on both sides of the transfer platform 2. For example, in the case where four incubators 1 are provided, two incubators 1 may be arranged on each side of the transfer platform 2. One part of the plurality of incubators 1 may be configured to carry out amplification culture of the biological samples, while another part of the plurality of incubators 1 may be configured to carry out simultaneous sedimentation enrichment of the biological samples. Thus, in the case where a plurality of incubators 1 are arranged, the individual incubators 1 may respectively take on the tasks of amplification culture and sedimentation enrichment as required.

The sample operating system 3 may be arranged as a whole in a controlled laminar flow environment, which allows the internal environment of the sample operating system 3 to be effectively separated from its external environment (e.g., the laboratory environment in which the sample operating system 3 is arranged). For example, as shown in FIG. 1, the sample operating system 3 may be provided with a laminar flow unit. In an embodiment according to the present disclosure, the laminar flow unit may be configured to form the laminar flow environment inside the sample operating system 3 using a pressure difference. The laminar flow unit may include a laminar flow air supply unit. The laminar flow air supply unit may be provided on top of the sample operating system 3. The laminar flow air supply unit may include an air supply device such as a blower or a centrifugal fan, which may deliver gases from an ambient or specialized gas source into the interior of the sample operating system 3 in a laminar flow. The laminar flow unit may also include a laminar flow air return unit. The laminar flow air return unit may be provided at the bottom of the sample operating system 3. The laminar flow air return unit can circulate and filter the laminar flow gas within the sample operating system 3 and circulate it back to the interior of the sample operating system 3, so that personnel, samples, and the surrounding environment can be effectively secured during operation. The laminar flow air supply unit and the laminar flow air return unit may be assigned with corresponding filtration units. By providing the laminar flow air supply unit and the laminar flow air return unit according to the present disclosure, the flow field in the operation area can be ensured to be uniform and consistent, and the flow rate is in accordance with the regulations, so as to ensure the safety of the production samples. At the same time. The system 10 according to the present disclosure can provide debugging function, so as to realize the working mode and fast exhaust mode according to the demand, to ensure to meet the national standards of biological safety cabinets and other national standards of wind speed and flow shape related requirements.

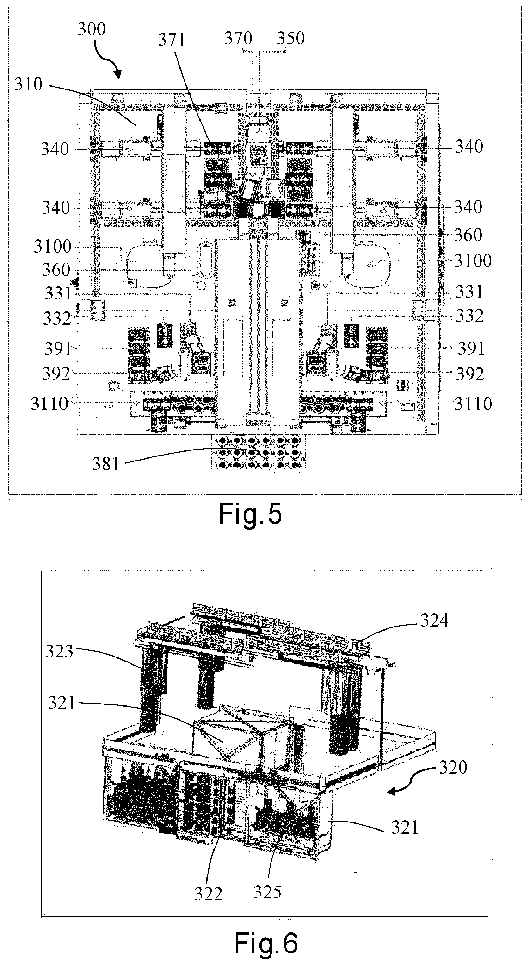

The sample operating system 3 according to an embodiment of the present disclosure is described in detail below with reference to FIGS. 5 to 13.

In an embodiment according to the present disclosure, the sample operating system 3 comprises an operating table surface 310 on which two sets of sample operating assemblies 300 may be mounted. The two sets of sample operating assemblies 300 may have the same arrangement and construction so as to be able to realize exactly the same functions. As described in FIG. 5, the two sets of sample operating assemblies 300 may be arranged on one side of the operating table surface 310 respectively. In this embodiment, the two sets sample operating assemblies 300 may be arranged mirror-symmetrically about a central axis of the operating table surface 310. The two sets of sample operating assemblies 300 may be configured to be capable of performing both independent operation and parallel operation, i.e. the two sets of sample operating assemblies 300 may be configured to be capable of not only operating independently of each other, but also of operating in parallel. By arranging such two sets of sample operating assemblies 300, the following advantages can be realized: 1) parallel operation of samples with different processes and different specification requirements can be realized, providing process adaptability; 2) the overall operating throughput of the system 10 according to the present disclosure can be increased multiplicatively; and 3) since two sets sample operating assemblies 300 are provided, for some of the critical operations in the system 10, it has a redundant design, which makes it possible to use the other set of sample operating assembly 300 to perform the corresponding critical operations in the event of failure of any one of the two sets of sample operating assemblies 300, thus avoiding the downtime of the whole system 10 and guaranteeing the high reliability of the automated operations of the whole system 10.

Referring to FIG. 5, in an embodiment according to the present disclosure, each sample operating assembly 300 may include at least one of the following devices: a code-scanning unit, a centrifuge tube shaking unit 330, a shake-flask shaking unit, a centrifuge tube uncapping unit 340, a shake-flask uncapping unit 3110, a magnetic bead sorting unit 360, a centrifuge unit 3100, a pipette carrier table 371, and a multi-degree-of-freedom robot 311. In addition, a pipetting unit 370 and a cryopreservation tube uncapping unit 350 are mounted on the operating table surface 310. The pipetting unit 370 and the cryopreservation tube uncapping unit 350 may be arranged on a central axis of the operating table surface 310. The operating table surface 310 together with the sample operating assembly 300 mounted on the operating table surface 310 may be arranged in an intermediate cabin. In embodiments according to the present disclosure, the sample operating system 3 may perform various operations for extracting macromolecules, including, but not limited to: scanning, uncapping, shaking, centrifugal separation, magnetic bead sorting, pipetting, pumping, and heating, etc. In this embodiment, the various devices in the sample operating system 3 are arranged in an island layout, and the following advantages can be realized through the island layout: 1) It is possible to realize the clustering of operations in each process link, so that for a specific operation, it is not necessary to transfer the biological sample back and forth between a plurality of different components, and instead, it is possible to complete the corresponding operation within the scope of the same device, thus improving the efficiency of the sample operation; 2) It is possible to multiply the single-unit operation throughput of the system 10 according to the present disclosure. A laminar flow air supply unit and a laminar flow air return unit can be arranged around the perimeter of the individual devices arranged according to an island layout, thereby ensuring a clean operating environment.

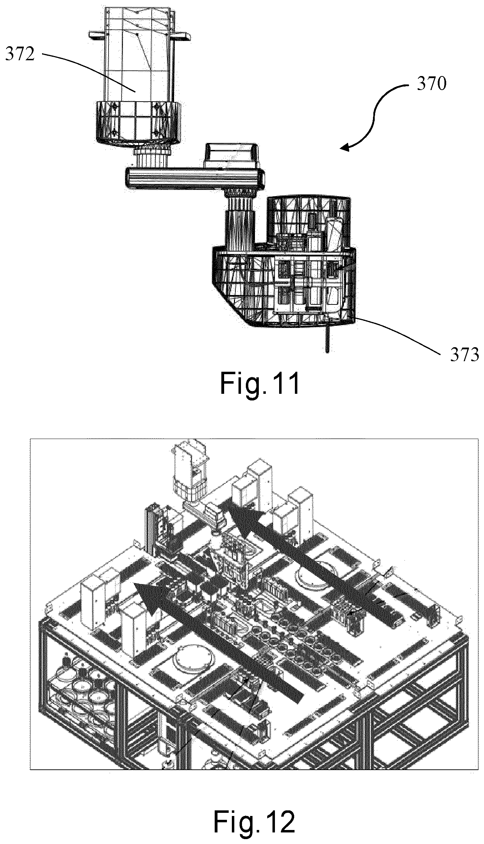

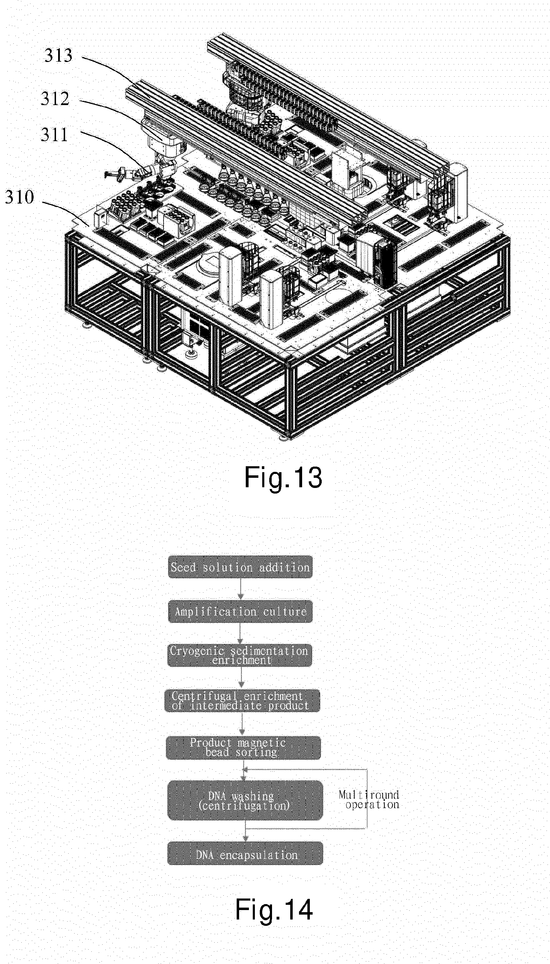

In an embodiment according to the present disclosure, in order to realize automated transfer and operation of consumables and biological samples within the sample operating system 3, a multi-degree-of-freedom robot 311 may also be provided on the operating table surface 310 of the sample operating system 3. The multi-degree-of-freedom robot 311 is capable of moving in a plurality of degrees of freedom (e.g., moving horizontally, moving vertically, rotating about its own central axis, deflecting about a horizontal axis and a vertical axis, etc.). In the embodiment shown in FIG. 13, the multi-degree-of-freedom robot 311 may be mounted on a linear motor module 312. The linear motor module 312 may include a transverse beam 313 that extends in a horizontal direction and a linear motor that is mounted on the transverse beam 313 and drives the multi-degree-of-freedom robot 311 in the direction of extension of the transverse beam 313. As shown in FIG. 12, the multi-degree-of-freedom robot 311 may move in the direction of the arrow. Of course, the multi-degree-of-freedom robot 311 may also move in a direction opposite to the arrow direction. As a result, the multi-degree-of-freedom robot 311 can move within a predetermined range of the sample operating system 3, thereby contributing to its automated transfer of consumables and biological samples within the predetermined range. The range of action of the multi-degree-of-freedom robot 311 is able to substantially cover the operating area of each device of the sample operating assembly 300. The setting of the multi-degree-of-freedom robot 311 helps to enhance the level of intelligence and automation of the system 10 according to the present disclosure. As shown in FIG. 13, for each sample operating assembly 300, there may be an assigned multi-degree-of-freedom robot 311 respectively. By setting up the multi-degree-of-freedom robot, a high-throughput and highly flexible transfer and operating unit may be obtained, whereby the following advantages may be realized: 1) efficient handling and transfer of different materials between different stations; 2) the ability to adjust and control the attitudes of different materials; and 3) The ability to perform anthropomorphic operations, such as waste liquid dumping;

According to an embodiment of the present disclosure, the code-scanning unit may be configured to identify an identification code, or a label element provided on the consumables to realize the recording and automatic management of the materials of the whole machine, and to realize the unified management of the database materials. The identification code may be configured as a bar code or a two-dimensional code. The label identification element may be configured as an RFID tag.

In an embodiment according to the present disclosure, as shown in FIG. 6, the sample operating system 3 may further comprise a pumping unit 320, the pumping unit 320 comprising a liquid storage unit 321 for storing liquid required in the process, a pump body panel 322 for selecting a channel for pumping the liquid, a pumping actuation module 323 for pumping liquids into consumables, and a motion module 324 for moving the pumping actuation module 323. The liquid can reach the pumping actuation module 323 from the liquid storage unit 321 via the pump body panel 322. The pumping actuation module can be configured as a centrifuge tube pumping device or a shake-flask pumping device. The pumping actuation module 323 may be mounted on the motion module 324 configured as a linear module. The motion module 324 may also comprise a transverse beam extending in a horizontal direction and a linear motor mounted on the transverse beam and driving the pumping actuation module 323 in the direction of extension of the transverse beam. As a result, the pumping actuation module 323 can move within a predetermined range of the sample operating system 3 to perform a liquid replenishment operation on a shake-flask, or consumable. The liquid storage unit 321 may be arranged, in one embodiment as shown in FIG. 6, partially in a lower region, such as a bottom cabin, of the sample operating system 3 located below the operating table surface 310, and partially in a region, such as an intermediate cabin, located above the operating table surface 310. In another embodiment, the liquid storage unit 321 may also be arranged entirely in a lower region of the sample operating system 3 located below the operating table surface 310, or entirely in a region located above the operating table surface 310. The liquid storage unit 321 can comprise at least one liquid storage container 325, such as a liquid storage bottle or a liquid storage tank. A weighing unit can be installed under the liquid storage container 325, by which the liquid remaining in the liquid storage container 325 can be weighed. In the event that the liquid remaining in the liquid storage container 325 falls below a specific threshold, this can be detected by the weighing unit and the superior equipment can be automatically notified to refill the liquid storage container 325. Alternatively or additionally, a level sensor may be provided in the liquid storage container 325, by which monitoring of the remaining liquid level may be realized. When the weighing unit or the level sensor detects that the remaining liquid is insufficient, an alarm may be issued to alert the refilling of the liquid storage container 325. In addition, the liquid storage container 325 may have a thermoregulator that may thermoregulate, e.g., heat, cool, or keep warm, the liquid stored in the liquid storage container 325, so that the stored liquid may have different liquid temperatures as desired. The pump body panel 322 may be arranged in a lower region of the sample operating system 3 located below the operating table surface 310. The pump body panel 322 may be arranged adjacent to the liquid storage unit 321. The pump body panel 322 may have a plurality of liquid pumping channels and is configured for selecting a liquid pumping channel among the plurality of liquid pumping channels. The plurality of liquid pumping channels may each be fluidly connected to a plurality of corresponding liquid storage containers 325, thereby forming a plurality of corresponding pump liquid supply lines. These pump fluid supply lines are independent of each other. In another embodiment, the pump body panel 322 can also have a single liquid pumping channel. The pumping actuation module 323 can move to different positions via the motion module 324 and perform a liquid pumping operation using the selected liquid pumping channel. The pumping unit 320 itself can have a liquid circuit cleaning function, which can realize quick cleaning, deep cleaning, reverse sanitization, and sanitization and cleaning of the inner and outer walls of the steel needle according to the needs of the use of the process liquid. In addition, the pumping unit 320 is assigned with a pump liquid heating unit which can heat the process fluid. The heating can be instantaneous. Process and cleaning requirements can thus be realized. The pumping unit 320 according to the present disclosure can realize the following advantages: 1) realizing the addition of large doses of solution in the production process, up to tens of ways to hundreds of ways of liquid pumping, each kind of liquid using a separate pump liquid line, no cross; 2) guaranteeing the cleanliness of the whole liquid operation circuit; 3) being able to monitor and alarm the liquid volume; 4) being able to achieve low, normal and high temperature control of the liquid stored in the liquid storage unit 321.

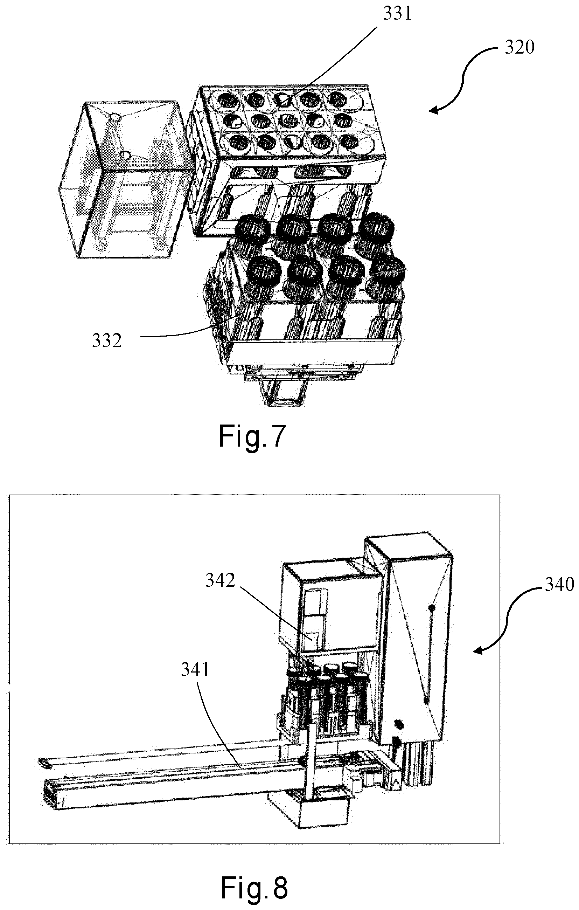

In an embodiment according to the present disclosure, as shown in FIG. 7, the centrifuge tube shaking unit 330 may be configured as a centrifuge tube oscillating and over-turning unit, which may comprise an over-turning unit 331 and a centrifuge tube oscillating unit 332. The centrifuge tube oscillating and over-turning unit may be configured to rotate the centrifuge tube at an angle of 0 degree to 360 degrees for mixing. At least one of the following parameters can be controlled during the mixing process: the rotation speed, the number of rotation cycles, and the direction of rotation. For example, the over-turning unit 331 can be made to over-turn in a positive or negative direction, or in a clockwise direction or in a counterclockwise direction, during the mixing process. The shake-flask shaking unit is structurally similar to the centrifuge tube shaking unit 330. By providing the centrifuge tube shaking unit 330 as well as the shake-flask shaking unit, automatic shaking of the samples during the production process can be realized.

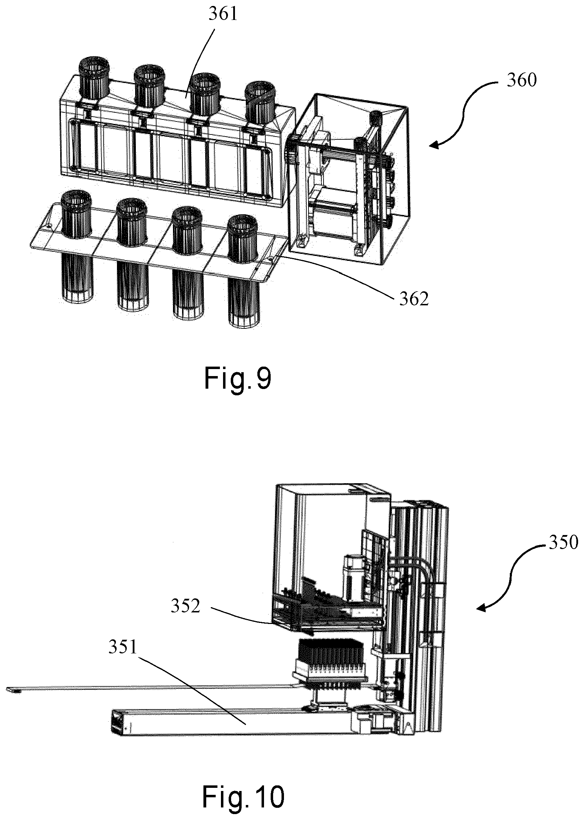

In an embodiment according to the present disclosure, as shown in FIG. 8, the centrifuge tube uncapping unit 340 may comprise a centrifuge tube loading linear module 341 and a centrifuge tube uncapping machine working head 342. Furthermore, the centrifuge tube loading linear module 341 may be assigned with a centrifuge tube automatic positioning and alignment mechanism. The centrifuge tube loading linear module 341 is configured to transport a centrifuge tube carrier tray underneath the centrifuge tube uncapping machine working head 342 after the multi-degree-of-freedom robot has placed the centrifuge tube carrier tray with centrifuge tubes on the centrifuge tube loading linear module 341. The centrifuge tube automatic positioning and alignment mechanism is configured to position and clamp the centrifuge tube carrier tray provided on the centrifuge tube loading linear module 341. As shown in FIG. 10, the cryopreservation tube uncapping unit 350 comprises a cryopreservation tube loading linear module 351 and a cryopreservation tube uncapping machine working head 352. In addition, the cryopreservation tube loading linear module 351 is assigned with a cryopreservation tube automatic positioning and alignment mechanism. The cryopreservation tube loading linear module 351 is configured to transport a cryopreservation tube carrier tray underneath the cryopreservation tube uncapping machine working head 352 after the multi-degree-of-freedom robot has placed the cryopreservation tube carrier tray on the cryopreservation tube loading linear module 351. The cryopreservation tube automatic positioning and alignment mechanism is configured for positioning and clamping the cryopreservation tube carrier tray provided on the cryopreservation tube loading linear module 351. The shake-flask automatic uncapping unit is configured similar to the centrifuge tube uncapping unit 340 or the cryopreservation tube uncapping unit 350. By providing the cryopreservation tube uncapping unit 350, the centrifuge tube uncapping unit 340, and the shake-flask automatic uncapping unit, automatic opening and closing of lids of various consumables can be realized, and the lids are stored using a special tooling that is placed upwards to avoid cross-materials.

In an embodiment according to the present disclosure, as shown in FIG. 9, the magnetic bead sorting unit 360 may comprise a magnetic bead sorting rack 361 for performing magnetic bead adsorption and a magnetic bead pouring rack 362 for dumping the waste supernatant liquid. The magnetic bead adsorption operation and the waste supernatant liquid dumping operation are capable of being performed simultaneously. By means of the automatic magnetic bead sorting unit 360, automatic sorting of sample liquid, automatic recovery of supernatant liquid, recovery of sorted magnetic beads, and automatic discharge of waste liquid can be realized. The magnetic bead sorting unit 360 can be assigned with a corresponding magnetic bead liquid shaking unit. The magnetic bead liquid shaking unit is configured to automatically shake the magnetic bead liquid, thereby realizing automatic shaking of the magnetic beads (sorting operation, etc.) prior to sample loading.

In an embodiment according to the present disclosure, the centrifuge unit 3100 may be configured as a temperature-controlled centrifuge unit 3100. The temperature-controlled centrifuge unit 3100 may function together with a corresponding material transfer unit to carry out an automatic low-temperature/normal-temperature/high-temperature centrifugation function of the samples and a material transfer to realize an effective mixing of the samples and collection of the effective products during the production process.

In an embodiment according to the present disclosure, as shown in FIG. 11, the pipetting unit 370 may be configured to transfer the liquid required in the process to a consumable placed on the pipette carrier table 371 to perform the corresponding operations. The pipetting unit 370 may comprise a pipetting robot 372 as well as a pipetting module 373. The pipetting module 373 is configured to be able to move independently within the pipetting unit 370 along a Z-axis, wherein the Z-axis is perpendicular to a horizontal plane. The pipetting robot 372 may be configured as a SCARA robot. By providing the pipetting unit 370 with independent movement in the Z-axis and mounting the pipetting module 373 on a SCARA robot platform, a greater range of pipetting operations can be realized and flexibility during operation can be greatly improved relative to conventional X-Y-Z linear motor pipetting platforms. In addition, the pipetting unit 370 allows for the addition of small doses of pharmaceuticals and the harvesting and encapsulation of the final product, etc.

In an embodiment according to the present disclosure, the sample operating system 3 may further comprise an operation carrier and a consumable carrier sliding table 381 for carrying the operation carrier, the consumable carrier sliding table 381 being configured to be capable of controlled sliding out of and into the sample operating system 3 to facilitate loading or unloading of consumables on the operation carrier.

In an embodiment according to the present disclosure, in addition to automatic loading, a manual loading table 391 may be provided by which manual loading can be performed. The manual loading table 391 is provided with a corresponding handling robot 392, wherein by the handling robot 392 the consumables can transfer into an automatic operation route after manual loading.

Figure 14 shows a schematic diagram of the sample operation process of the present invention. First the seed solution can be added to the consumables (e.g. shake-flasks). The consumables with the seed solution are then amplified and cultured in the incubator 1 to form an amplified and cultured biological sample. The amplified and cultured biological sample is then enriched by cryogenic sedimentation in the incubator 1. The amplification culture and cryogenic sedimentation enrichment may be performed in the same incubator 1 or in different incubators 1 in the incubator array. The biological sample is then transferred from the incubator 1 to the sample operating system 3 via the transfer platform 2. Corresponding operations are performed on the biological samples in the sample operating system 3. After centrifugal separation and enrichment of the biological samples, intermediate products can be obtained. In order to obtain a sufficient amount of the intermediate product, liquid replenishment can be performed again, and the replenished biological sample can be transferred from the sample operating system 3 to the incubator 1 via the transfer platform 2, and amplification culture and cryogenic sedimentation enrichment can be performed again. After completion of the centrifugal enrichment of the desired intermediate products, the products can be subjected to magnetic bead sorting. Next, DNA cleaning can be performed, e.g., by centrifugation, which can be performed in multiple rounds. Finally, DNA encapsulation is performed, thereby completing all steps of the extraction of macromolecules.

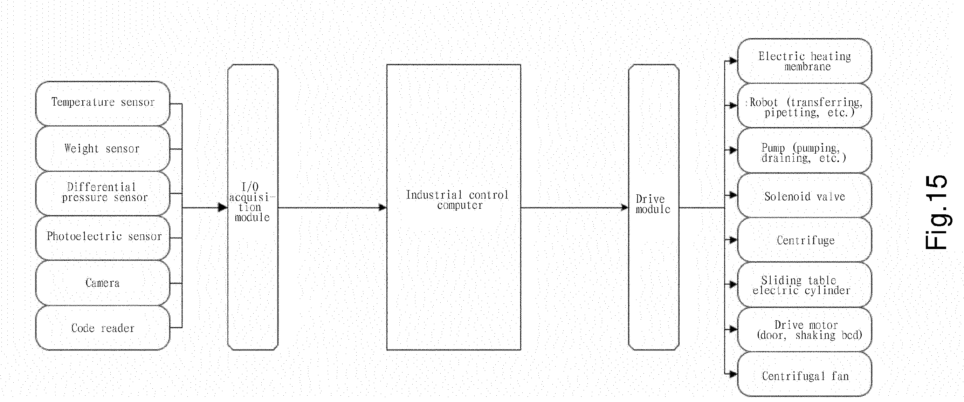

As described above, in an embodiment according to the present disclosure. The system 10 may be configured as an automated and intelligent system, and all operations of the modules in the system 10 (e.g., the incubator 1, the sample operating system 3, the transfer platform 2, etc.) may be completed automatically under the control of an automated control system. FIG. 15 illustrates an architecture of the automated control system 6 according to an embodiment of the present disclosure. As shown in FIG. 15, the automated control system 6 according to the present disclosure may comprise an industrial computer, an input/output data acquisition module, and a drive module. For data acquisition, various acquisition devices may be provided within the system 10, including but not limited to: temperature sensors, weight sensors, differential pressure sensors, photoelectric sensors, cameras, code readers, etc. Among them, the temperature sensor, the weight sensor, the differential pressure sensor, the photoelectric sensor, the camera, and the like may be arranged at appropriate locations within the system 10 as required. The drive module is used to control the operation of various components or modules within the system 10, including, but not limited to, the operation of the following components or modules: electric heating membranes, robots (transferring, pipetting, etc.), pumps (pumping, draining, etc.), solenoid valves, centrifuges, sliding table electric cylinders, drive motors (e.g., drive motors for the doors of the incubators and for the shaking beds), centrifugal fans, and the like.

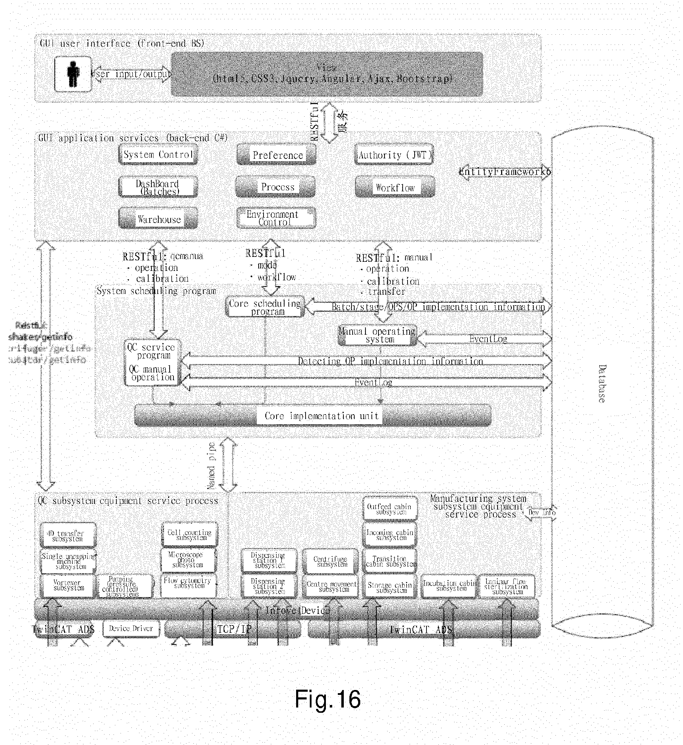

FIG. 16 illustrates complete full process control software accompanying a system according to an embodiment of the present disclosure. At least one of the following functions can be realized by the control software in the system 10 of the present disclosure: a process editing function, an equipment operation monitoring and operation function (for the fully automated operation mode), an equipment debugging work function (for the manual operation mode), a logging reminder function, and a setup function. The process editing function includes: editing and management of the whole process list; provision of standardized process templates; and real-time process control and adjustment. Equipment operation monitoring and operation functions include: sample liquid operation history data; sample magnetic bead operation history data; sample centrifugation operation history data; sample culture operation history data; sample transfer operation history data; material status management at each station; monitoring of the operation status of each equipment module; solid consumable sampling management; liquid material sampling management; storage center consumable storage management; storage center consumable replenishment management; and culture whole column shake-flasks status management. Equipment debugging (manual operation mode) includes: calibration of each motion module in the system; calibration of each liquid unit in the system; log prompt function; recording of operator login information; material loading information; recording of equipment failure information during operation; recording of equipment warning information during operation; and recording of equipment operation information during operation. The setup function includes: personnel authority setting; parameter setting for each module of the system; liquid line type configuration; and liquid capacity configuration.

With the system of the present disclosure, the following advantages can be obtained:
(1) realizing a throughput that is difficult to reach by manual operation in the research and development and production stage, and realizing a throughput increase of two orders of magnitude. Configuring a full-process intelligent scheduling system to maximize equipment and facility throughput and eliminate waiting waste in the process;
(2) Effectively avoid cross contamination of different batches of samples in the culture process during large-scale production. Continuously maintain high cleanliness of samples and environment during the production process;
(3) The whole process of anthropomorphic operation and match the molecular extraction of common process modules, high flexibility to match the needs of different process operations;
(4) Flexible process editing capabilities, and save common process flow within the system software, to achieve high-speed switching of processes in the research and development stage;
(5) Continuous collection, storage and analysis of process parameters and process results during the process to achieve online optimization of the process;
(6) Flexible adjustment of different production scales;
(7) Using high-throughput fully automated temperature-controlled incubator arrays to realize large-scale cultivation of seed bacterial fluids, and providing expansion interfaces for rapid scale-up;
(8) Realization of single day hundred/thousand/ten thousand level culture of units with different sequence design plasmid culture vectors.

Other embodiments of the present disclosure are disclosed below.

Referring to FIGS. 1-16, the present invention provides a technical solution:
An automated production system suitable for the extraction of macromolecules, comprising a top cabin, an intermediate cabin, a bottom cabin, a temperature-controlled incubator, a robot and a rail, a core operating system and an electrical control system, the top cabin comprising a laminar flow air intake and a robot rail, the intermediate cabin comprising a code-scanning unit, an uncapping unit, a shaking unit, a centrifuge, a magnetic bead sorting unit, a pipetting unit, a pump-liquid storage unit and a pump body unit, a heating unit, an electrically operated door and a manual door module. The bottom cabin comprises a liquid storage cabin, a centrifugal equipment storage cabin, a pump valve control cabin, an air return blower and a cable liquid circuit.

An equipment frame unit is provided within the temperature-controlled incubator, wherein a shaking bed mechanism driving unit is provided on the lower side of the interior of the temperature-controlled incubator, wherein an automatic sliding table unit is provided in the interior of the temperature-controlled incubator and located on the upper side of the shaking bed mechanism driving unit, wherein a shake-flask carrier tray unit is provided on the upper side of the automatic sliding table unit, wherein an equipment lighting/purification/sanitization unit is provided on the upper side of the interior of the temperature-controlled incubator, wherein an intelligent integrated control unit is provided on the right rear side of the temperature-controlled incubator, wherein an equipment operation and display area is provided on the right side of the temperature-controlled incubator, wherein an automatic sealing door unit is provided on the right side of the end of the temperature-controlled incubator.

The core operating system includes a loading sliding table, a pipette carrier table, a cryopreservation tube uncapping unit, a pipetting robot and module, a centrifuge tube uncapping unit, a centrifuge, a magnetic bead sorting unit, a centrifugal over-turning unit, a centrifuge tube oscillating unit, a manual loading table, a handling robot, and an automatic loading table for shake-flasks to open the caps.

The pump liquid storage and pump body unit includes large capacity liquid storage, multi-channel pump body panel, pump liquid execution unit linear motion module, pumping execution module.

The cryopreservation tube uncapping unit and centrifuge tube uncapping unit both include loading linear module, uncapping machine working head, and automatic positioning and alignment mechanism.

The robot independently moves the pipetting unit along the Z-axis, and is mounted on a SCARA robot platform.

The electrical control system includes temperature sensors, weight sensors, differential pressure sensors, photoelectric sensors, cameras, code readers, I/O acquisition modules, industrial control computer and drive modules.

Examples of implementation: a) Core operating system: based on the multi-robot system with full automation module, to realize the replacement of manual operation in the whole process of the macromolecules extraction;
1~2 orders of magnitude to enhance the productivity of the macromolecules extraction process link (20~30 samples/day processing throughput for manual team, hundreds to thousands of samples/day processing throughput for automated system); the whole operation process is sealed in laminar flow structure to isolate the risk of environmental contamination to the samples, and at the same time, isolate the risk of potential biological contamination of samples in the system to the installation environment;
b) Large-scale temperature-controlled shake-flasks incubator array:
   - Expansion of plasmid culture vector cells in a heated incubator;
   - Effective sedimentation enrichment of vector cells in a low-temperature incubator to facilitate replenishment and exchange operations in the core operating system;
c) Transfer unit:
   - Robot and accompanying rail system to realize the efficient transfer of samples between the core operating system and the incubator array;

Fully automated incubator unit:
1) Realize fully-automatic temperature and humidity controlled culture (25~38C temperature control, 70~95% humidity control);
2) Realize fully-automatic low-temperature sample sedimentation enrichment (2C~8C temperature control for sample sedimentation);
3) Realize fully-automatic slide-in and slide-out operation of samples, and docking with fully-automatic robot transfer system;
4) Realize fully-automatic opening and closing of the incubator door, and docking with the fully automatic robot transfer system;
5) Intelligent network unit to realize remote control of incubator array by factory software;

After the incubator array obtains the culture consumables (such as shake-flasks, etc.), the first batch is placed on the transit sliding table, and after the single batch is placed, the batch is transferred to the core liquid operating system, which can greatly improve the transfer efficiency; after the shake-flasks complete the replenishment/liquid exchange operation, the batch is transferred to the sliding table, and uniformly transferred back to the incubator array;

Example of the complete operation flow of the system.
Step1. The incubator door opens automatically, the sliding table slides out, and the transfer robot transfers the culture samples (shake-flasks) to the transfer platform;
Step2. The transfer platform transfers the batch of culture samples to the core operating system and completes the loading to the interior of the core operating system;
Step3. The core operating system carries out sample operation and completes data acquisition and analysis in the process;
Step4. The culture samples are transferred from the core operating system to the sliding table;
StepS. The incubator door opens automatically, the slide table slides out, and the robot feeds into the incubator to resume culture;

Core operating system:
(1) The left and right sides of the system are arranged in mirror image, and the left and right sides run in parallel to improve the overall operating throughput and facilitate emergency redundancy backup;
(2) each work unit is laid out in an island type, and each process link is operated in a clustered way to improve the throughput of single-step operation;
(3) Laminar air inlet and return air circuits are arranged around the island of each working unit to ensure a clean operating environment;

The core operating system contains core modules:
(1) Automatic loading platform, loading temporary storage and operation disk group disk;
(2) High-degree-of-freedom flexible robot unit (including linear guide), for various types of material handling and anthropomorphic operation;
(3) Pumping unit for pumping large-capacity liquids and fully automatic cleaning of lines; Pump liquid storage and pump unit array
   - includes: large-capacity liquid storage, multi-channel pump body panel, pump liquid execution unit linear motion module, pumping execution module;
   - can perform pumping operation of selected channels in any position under the linear motion module; under each liquid storage bottle, a weighing unit is installed for automatic notification of liquid replenishment.
(4) Centrifuge tube oscillating and over-turning unit for sample mixing in centrifuge tubes; Realizes efficient rotary mixing of the centrifuge tube array from 0 to 360 degrees, and controls the rotary speed, number of rotary cycles, and positive and negative directions during mixing;
(5) Centrifuge tube uncapping unit: including: loading linear module, uncapping machine working head, automatic positioning and alignment mechanism; after the robot transfers the centrifuge tube tray to the linear module, the positioning mechanism performs automatic clamping and alignment operation;
(6) Magnetic bead sorting unit: while adsorbing magnetic beads, automatically dumping the waste supernatant;
(7) Cryopreservation tube uncapping unit: including: loading linear module, uncapping machine working head, automatic positioning and alignment mechanism; after the robot transfers the centrifuge tube tray to the linear module, the positioning mechanism performs automatic clamping and alignment operation;
(8) Temperature-controlled centrifuge unit;
(9) Pipetting platform;
(10) Pipetting unit; which independently moves the pipetting unit along the Z-axis, and is installed on the SCARA robot platform; compared with the traditional X-Y-Z linear motor pipetting platform, it realizes a larger range of pipetting operations and greatly improves the flexibility in the operation process;
(11) Side view of the basic structure of the core operating system, the working table surface is optimized to achieve a near unidirectional material flow, further improving the throughput and efficiency of the production line;

1. The size of the incubator array can be adjusted to increase or decrease in number based on actual process needs;
2. Other culture methods such as bit reactor array can be replaced;
3. The core operating system can be increased in number based on actual process requirements and interfaced with a fully automated culture system;
4. Based on the actual process requirements, the types and quantities of robot units, various types of consumable uncapping units, bead handling units, centrifuge units, pumping units, pipetting units, etc., can be adjusted within the core liquid operating system;
5. Based on the actual process requirements, the internal layout of the core system is adjusted;

A complete example process of molecular extraction: as shown in Fig. 14; the core operating system is configured with a complete electrical control system to realize the automated operation of the whole process: as shown in Fig. 15; the system is accompanied by a complete full process control software: as shown in Fig. 16.

Although embodiments of the present invention have been shown and described, it will be appreciated by those of ordinary skill in the art that a variety of changes, modifications, substitutions and variations may be made to these embodiments without departing from the principles and spirit of the present invention, the scope of which is limited by the appended claims and their equivalents.

## Claims

1. A system for the extraction of macromolecules, comprising:
at least one incubator for culturing a biological sample to provide a base sample for the extraction of the macromolecules;
a sample operating system for performing predetermined operations on the biological sample cultured by the incubator to extract macromolecules from the base sample;
a transfer platform for transferring the biological sample between the incubator and the sample operating system.

2. The system for the extraction of macromolecules according to claim 1, wherein the transfer platform comprises a robot.

3. The system for the extraction of macromolecules according to claim 2, wherein the robot is provided on a ground rail or an air rail.

4. The system for the extraction of macromolecules according to claim 2, wherein the transfer platform comprises a transit sliding table, the robot being capable of transferring the biological sample from the incubator to the transit sliding table, the transit sliding table being configured for transferring the biological sample to the sample operating system.

5. The system for the extraction of macromolecules according to claim 2, wherein the transit sliding table is capable of transferring biological samples in batch.

6. The system for the extraction of macromolecules according to claim 1, wherein a plurality of incubators is provided, the plurality of incubators being arranged on both sides of the transfer platform.

7. The system for the extraction of macromolecules according to claim 1, wherein the incubator comprises a housing and a culture assembly accommodated within the housing, the culture assembly being provided on an automatic sliding table element to enable the culture assembly to be slid out of or into the housing in a controlled manner.

8. The system for the extraction of macromolecules according to claim 7, wherein the incubator is configured to conduct automatic temperature control and humidity control of the biological sample, wherein the internal temperature of the incubator is controlled at 15 to 40°C, and the humidity thereof is controlled at 70 to 95%, for amplification culture.

9. The system for the extraction of macromolecules according to claim 8, wherein the incubator comprises a heating membrane, wherein the heating membrane automatically heats the incubator and controls the internal temperature thereof at 15 to 40°C.

10. The system for the extraction of macromolecules according to claim 8, wherein the incubator comprises a humidification unit, the humidification unit comprising an automatic water supply unit and a nebulizer, the automatic water supply unit being configured to supply water to the incubator in a controlled manner, and the nebulizer being configured to control the humidity of the incubator by nebulizing the water from the automatic water supply unit.

11. The system for the extraction of macromolecules according to claim 7, wherein the incubator comprises a refrigeration compressor, the refrigeration compressor being configured to control the temperature of the biological samples at 2 to 8°C when sedimentation enrichment of the biological sample is required.

12. The system for the extraction of macromolecules according to claim 7, wherein a portion of the incubators is configured to carry out an amplification culture of the biological samples, and another portion of the incubators is configured to simultaneously carry out a sedimentation enrichment of the biological samples.

13. The system for the extraction of macromolecules according to claim 1, wherein the sample operating system is arranged in a controlled laminar flow environment such that the internal environment of the sample operating system is isolated from its external environment.

14. The system for the extraction of macromolecules according to claim 13, wherein the sample operating system is provided with a laminar flow unit, the laminar flow unit being configured to form the laminar flow environment inside the sample operating system using a pressure difference.

15. The system for the extraction of macromolecules according to claim 14, wherein the laminar flow unit comprises a laminar flow air supply unit and a laminar flow air return unit, the laminar flow air supply unit being arranged at the top of the sample operating system and the laminar flow air return unit being arranged at the bottom of the sample operating system.

16. The system for the extraction of macromolecules according to claim 1, wherein the sample operating system comprises an operating table surface, on which two sets of sample operating assemblies are mounted, the two sets of sample operating assemblies being arranged mirror-symmetrically about a central axis of the operating table surface on one side of the operating table surface respectively, and the two sets of sample operating assemblies being configured to be capable of carrying out both independent operation and parallel operation.

17. The system for the extraction of macromolecules according to claim 16, wherein the sample operating assembly comprises at least one of the following devices: a code-scanning unit, a centrifuge tube shaking unit, a shake-flask shaking unit, a centrifuge tube uncapping unit, a shake-flask uncapping unit, a magnetic bead sorting unit, a centrifuge unit, a pipette carrier table, and a multi-degree-of-freedom robot.

18. The system for the extraction of macromolecules according to claim 17, wherein a pipetting unit and a cryopreservation tube uncapping unit are further mounted on the operating table surface, the pipetting unit and the cryopreservation tube uncapping unit being arranged on a central axis of the operating table surface.

19. The system for the extraction of macromolecules according to claim 16, wherein the sample operating system is configured to perform at least one of the following operations: scanning, uncapping, shaking, centrifugal separation, magnetic bead sorting, pipetting, pumping and heating.

20. The system for the extraction of macromolecules according to claim 17, wherein the multi-degree-of-freedom robot is mounted on a linear motor module, the linear motor module comprising a transverse beam extending in a horizontal direction and a linear motor mounted on the transverse beam and driving the multi-degree-of-freedom robot in the direction of extension of the transverse beam, the multi-degree-of-freedom robot being capable of moving on the transverse beam such that the range of action of the multi-degree-of-freedom robot is capable of substantially covering the operating area of each device of the sample operating assembly.

21. The system for the extraction of macromolecules according to claim 16, wherein the sample operating system further comprises a pumping unit, the pumping unit comprising a liquid storage unit for storing liquids required for the process, a pump body panel for selecting a liquid pumping channel, a pumping actuation module for pumping liquids into consumables, and a motion module for moving the pumping actuation module.

22. The system for the extraction of macromolecules according to claim 21, wherein the pump body panel is configured for selecting a liquid pumping channel among a plurality of liquid pumping channels, the pumping actuation module being able to move to different positions by the motion module and to perform a liquid pumping operation using the selected liquid pumping channel.

23. The system for the extraction of macromolecules according to claim 21, wherein the liquid storage unit comprises at least one liquid storage container.

24. The system for the extraction of macromolecules according to claim 23, wherein a weighing unit can be installed under the liquid storage container, by which the liquid remaining in the liquid storage container can be weighed.

25. The system for the extraction of macromolecules according to claim 21, wherein the pumping actuation module is configured as a centrifuge tube pumping device or a shake-flask pumping device.

26. The system for the extraction of macromolecules according to claim 17, wherein the centrifuge tube shaking unit is configured as a centrifuge tube oscillating and over-turning unit which is configured to rotate the centrifuge tube at an angle at 0 degree to 360 degrees for mixing, wherein at least one of the following parameters can be controlled during the mixing process: rotation speed, number of rotation cycles, and direction of rotation.

27. The system for the extraction of macromolecules according to claim 17, wherein the centrifuge tube uncapping unit comprises a centrifuge tube loading linear module and a centrifuge tube uncapping machine working head, the centrifuge tube loading linear module being configured to transport a centrifuge tube carrier tray underneath the centrifuge tube uncapping machine working head after the multi-degree-of-freedom robot has placed the centrifuge tube carrier tray with centrifuge tubes on the centrifuge tube loading linear module.

28. The system for the extraction of macromolecules according to claim 27, wherein the centrifuge tube loading linear module is assigned with a centrifuge tube automatic positioning and alignment mechanism, the centrifuge tube automatic positioning and alignment mechanism being configured to position and clamp the centrifuge tube carrier tray.

29. The system for the extraction of macromolecules according to claim 17, wherein the magnetic bead sorting unit comprises a magnetic bead sorting rack for performing magnetic bead adsorption and a magnetic bead pouring rack for dumping waste supernatant.

30. The system for the extraction of macromolecules according to claim 29, wherein the magnetic bead adsorption operation and the waste supernatant pouring operation can be performed simultaneously.

31. The system for the extraction of macromolecules according to claim 18, wherein the cryopreservation tube uncapping unit comprises a cryopreservation tube loading linear module and a cryopreservation tube uncapping machine working head, the cryopreservation tube loading linear module being configured to transport a cryopreservation tube carrier tray underneath the cryopreservation tube uncapping machine working head after the multi-degree-of-freedom robot has placed the cryopreservation tube carrier tray on the cryopreservation tube loading linear module.

32. The system for the extraction of macromolecules according to claim 31, wherein the cryopreservation tube loading linear module is assigned with a cryopreservation tube automatic positioning and alignment mechanism, the cryopreservation tube automatic positioning and alignment mechanism being configured to position and clamp the cryopreservation tube carrier tray.

33. The system for the extraction of macromolecules according to claim 18, wherein the pipetting unit is configured to transfer the liquid required in the process to a consumable placed on the pipette carrier table to perform the corresponding operation.

34. The system for the extraction of macromolecules according to claim 33, wherein the pipetting unit comprises a pipetting robot as well as a pipetting module, the pipetting module being configured to be capable of moving independently within the pipetting unit along a Z-axis, wherein the Z-axis is perpendicular to a horizontal plane.

35. The system for the extraction of macromolecules according to claim 34, wherein the pipetting robot is configured as a SCARA robot.

36. The system for the extraction of macromolecules according to claim 16, wherein the sample operating system further comprises an operation carrier and a consumable carrier sliding table for carrying the operation carrier, the consumable carrier sliding table being configured to be capable of sliding out of and into the sample operating system in a controlled manner so as to facilitate loading or unloading of consumables on the operation carrier.

37. The system for the extraction of macromolecules according to claim 16, wherein the operating table surface together with the sample operating assembly mounted on the operating table surface is arranged in an intermediate cabin.

38. The system for the extraction of macromolecules according to claim 21, wherein the liquid storage unit and the pump body panel are arranged in a bottom bin.

39. The system for the extraction of macromolecules according to claim 1, wherein the system for the extraction of macromolecules further comprises an automated control system, the automated control system being configured to control operations in the incubator, the sample operating system, and the transfer platform.
